# EUROPEAN PATENT APPLICATION

(11) **EP 3 400 972 A1**
(43) Date of publication of application: **14.11.2018**
(21) Application number: 16883795.3
(22) Date of filing: 21.12.2016
(51) Int. Cl.: A61L 27/14, A61K 9/06, A61K 47/34, C08G 65/329

(54) **GEL MATERIAL FOR OPHTHALMIC TREATMENT USE**

(30) Priority: 06.01.2016 JP 2016000913
(71) Applicant: The University Of Tokyo, Tokyo 113-8654 (JP); University of Tsukuba, Tsukuba-shi, Ibaraki 305-8577 (JP)
(72) Inventor: SAKAI, Takamasa, Tokyo 113-8654 (JP); TEI, Yuichi, Tokyo 113-8654 (JP); OKAMOTO, Fumiki, Tsukuba-shi Ibaraki 305-8577 (JP); HOSHI, Sujin, Tsukuba-shi Ibaraki 305-8577 (JP)
(74) Representative: Patronus IP Patent- und Rechtsanwälte
(86) International application number: PCT/JP2016/088111
(87) International publication number: WO 2017/119296

(57) **Abstract**

Provided is a gel material for ophthalmic treatment useful as a synthetic vitreous body which is a novel intraocular tamponade material having a low swelling pressure, an appropriate elastic force, and no toxicity to ocular tissues, specifically, to retinas, and which is capable of stably maintaining a long-term stable tamponade effect.

A gel material for ophthalmic treatment including a hydrogel in which a gel precursor cluster crosslinks to form a three-dimensional network. The gel precursor cluster has a structure with crosslinked monomer units or crosslinked polymer units present at concentrations less than a critical gelation concentration, and the gel precursor cluster has a relationship of G' < G" where G' represents a storage elastic modulus and G" represents a loss elastic modulus. The hydrogel has a polymer content of 50 g/L or less, a storage elastic modulus G' of 1 to 10,000 Pa at a frequency of 1 Hz, and a fractal dimension of 1.5 to 2.5.

## Description

### Technical Field

The present invention relates to a gel material for ophthalmic treatment which is useful as a biomaterial such as a synthetic vitreous body and has a low swelling pressure, an appropriate elastic force, and no cytotoxicity, and relates to a polymer composition for forming the gel material.

### Background Art

The vitreous body, which is at the back of the crystalline lens of an eyeball, is a clear, colorless gel-like material covering most of the volume of eyeball. In recent years, due to development of intraocular laser technology, surgeries such as vitrectomy have been performed for the treatment of disorders such as macular hole, retinal detachment, and proliferative vitreoretinopathy. During such vitreous surgeries, it is required to inject gas or liquid that occupies a considerable volume in a vitreous cavity, or a closed cavity, as a replacement material for the resected vitreous body (what is called an intraocular tamponade material) so as to press retinas from the inside of the eyeball and to prevent detachment of the retinas.

In the related art, examples of such an intraocular tamponade material include gas such as the air, SF₆ gas, and C₃F₈ gas, or liquid such as silicone oil, and perfluorocarbon. However, in using a gas tamponade material, since the effect of the gas tamponade material is temporary due to intraocular gas absorption, retinas cannot be pressed for a long period of time (Patent Literature 1 and the like). On the other hand, in using a liquid tamponade material, it is required to remove the liquid tamponade material after surgery or after a certain period of time because of its high toxicity to ocular tissues, so that handling of the liquid tamponade material is troublesome. In addition, when using these intraocular tamponade materials, a patient is forced to lie down prone usually for about a week after surgery, which places a heavy burden on the patient, and what is more, pressure cannot be sufficiently controlled by the tamponade materials, which raises concern about the onset of cataract due to an increase in intraocular pressure.

As a new tamponade material, there is a proposal on a composition including polyethylene glycol that has an end modified by a long-chain alkyl group (for example, Patent Literature 2). However, this composition has extremely high hardness and applies a heavy burden on an eyeball due to its necessity of using a needle (21 gauge) thicker than a typically-used injection needle (25 gauge), which may lead to an appreciable period for treatment. Technology using a hydrogel such as hyaluronic acid and polyvinyl alcohol has also been studied but is still impractical due to problems such as inability to control swelling after a long period of time.

### Citation List

### Patent Literature

Patent Literature 1: JP 5-184663 A
Patent Literature 2: JP 2010-104632 A

### Summary of Invention

### Technical Problem

In light of such problems in the related art, an object of the present invention is to provide a gel material for ophthalmic treatment useful as a synthetic vitreous body, or a novel intraocular tamponade material having a low swelling pressure, an appropriate elastic force, and no toxicity to ocular tissues, specifically, to retinas, and being capable of stably maintaining a long-term stable tamponade effect.

### Solution to Problem

As a result of intensive studies to achieve the above object, the present inventors have found the following facts in regard to a hydrogel at low polymer concentration obtained when using, as species in a gelation reaction, gel precursor clusters which are intentionally put in a state on the verge of gelation, more specifically, in a sol state where a storage elastic modulus G' is smaller than a loss elastic modulus G": the fact that the hydrogel may function as an intraocular tamponade material and a synthetic vitreous body having a low swelling pressure stable for a long period of time and having an appropriate elastic modulus; and the fact that the gel precursor clusters may be injected into a living body in a solution state in a minimal invasive approach and allowed to gel in vivo so as to be self-assembled, thereby completing the present invention.

In other word, an aspect of the present invention provides,
(1) a gel material for ophthalmic treatment including a hydrogel in which a gel precursor cluster crosslinks to form a three-dimensional network,
   wherein the gel precursor cluster has a structure with a crosslinked monomer unit or a crosslinked polymer unit present at a concentration less than a critical gelation concentration, and the gel precursor cluster has a relationship of G' < G" where G' represents a storage elastic modulus and G" represents a loss elastic modulus, and
   wherein the hydrogel has a polymer content of 50 g/L or less, a storage elastic modulus G' of 1 to 10,000 Pa at a frequency of 1 Hz, and a fractal dimension of 1.5 to 2.5.

A preferred aspect of the gel material for ophthalmic treatment of the present invention provides:
(2) the gel material for ophthalmic treatment according to (1), wherein the hydrogel has a loss elastic modulus G" of 1 to 100 Pa;
(3) the gel material for ophthalmic treatment according to (1) or (2), wherein, in an aqueous solution, the hydrogel has a swelling pressure of 0.1 to 5 kPa and a swelling degree in a range where the volume of the hydrogel in a temperature of 30 to 40°C changes from 90 to 500% of the volume at the time of gel formation;
(4) the gel material for ophthalmic treatment according to any one of (1) to (3), wherein the monomer unit has a vinyl skeleton, or the polymer unit has a polyethylene glycol skeleton or a polyvinyl skeleton;
(5) the gel material for ophthalmic treatment according to any one of (1) to (4), wherein the gel precursor cluster includes a first polymer unit having one or more nucleophilic functional groups in a side chain or at an end and a second polymer unit having one or more electrophilic functional groups in a side chain or at an end;
(6) the gel material for ophthalmic treatment according to (5), wherein the nucleophilic functional group is selected from the group consisting of an amino group, - SH, and -CO₂PhNO₂, and the electrophilic functional group is selected from the group consisting of N-hydroxy-succinimidyl (NHS) group, a sulfosuccinimidyl group, a maleimidyl group, a phthalimidyl group, an imidazoyl group, an acryloyl group, and a nitrophenyl group;
(7) the gel material for ophthalmic treatment according to (5), wherein the nucleophilic functional group is -SH, and the electrophilic functional group is a maleimidyl group;
(8) the gel material for ophthalmic treatment according to (5), wherein the gel precursor cluster includes a first gel precursor cluster and a second gel precursor cluster, wherein the first gel precursor cluster has a content of the first polymer unit higher than a content of the second polymer unit, and the second gel precursor cluster has a content of the second polymer unit higher than a content of the first polymer unit;
(9) the gel material for ophthalmic treatment according to any one of (1) to (8), wherein the loss elastic modulus G" of the gel precursor cluster is in the range of 0.005 to 5 Pa at a frequency of 1 Hz;
(10) the gel material for ophthalmic treatment according to any one of (1) to (9), wherein the gel precursor cluster has a fractal dimension of 1.5 to 2.5;
(11) the gel material for ophthalmic treatment according to any one of (1) to (10), wherein the gel precursor cluster has a diameter in the range of 10 to 1000 nm;
(12) the gel material for ophthalmic treatment according to any one of (1) to (11), wherein the gel material is used as a vitreous injectant; and
(13) the gel material for ophthalmic treatment according to any one of (1) to (11), wherein the gel material is used as a synthetic vitreous body.

In another aspect, the present invention relates to a polymeric composition for ophthalmic treatment including a gel precursor cluster, providing:
(14) a polymer composition for ophthalmic treatment including a gel precursor cluster, wherein the gel precursor cluster has a structure with a crosslinked monomer unit or a crosslinked polymer unit at a concentration less than a critical gelation concentration, and the gel precursor cluster has a relationship of G' < G" where G' represents a storage elastic modulus and G" represents a loss elastic modulus;
(15) the polymer composition for ophthalmic treatment according to (14), wherein the monomer unit has a vinyl skeleton, or the polymer unit has a polyethylene glycol skeleton or a polyvinyl skeleton;
(16) the polymer composition for ophthalmic treatment according to (14) or (15), wherein the gel precursor cluster includes a first polymer unit having one or more nucleophilic functional groups in a side chain or at an end and a second polymer unit having one or more electrophilic functional groups in a side chain or at an end;
(17) the polymer composition for ophthalmic treatment according to (16), wherein the nucleophilic functional group is selected from the group consisting of an amino group, -SH, and -CO₂PhNO₂, and the electrophilic functional group is selected from the group consisting of N-hydroxy-succinimidyl (NHS) group, a sulfosuccinimidyl group, a maleimidyl group, a phthalimidyl group, an imidazoyl group, an acryloyl group, and a nitrophenyl group;
(18) the polymer composition for ophthalmic treatment according to (16), wherein the nucleophilic functional group is -SH, and the electrophilic functional group is a maleimidyl group;
(19) the polymer composition for ophthalmic treatment according to (16), wherein the gel precursor cluster has a content of the first polymer unit higher than a content of the second polymer unit, or the gel precursor cluster has a content of the second polymer unit higher than a content of the first polymer unit;
(20) the polymer composition for ophthalmic treatment according to any one of (14) to (19), wherein the gel precursor cluster has the loss elastic modulus G" in the range of 0.005 to 5 Pa at a frequency of 1 Hz;
(21) the polymer composition for ophthalmic treatment according to any one of (14) to (20), wherein the gel precursor cluster has a fractal dimension of 1.5 to 2.5; and
(22) the polymer composition for ophthalmic treatment according to any one of (14) to (21), wherein the gel precursor cluster has a diameter in the range of 10 to 1000 nm.

In another aspect, the present invention relates to a kit including a polymer composition for ophthalmic treatment, providing:
(23) a kit including the polymer composition for ophthalmic treatment according to any one of (16) to (22);
(24) the kit according to (23), further including a crosslinking agent.
(25) the kit according to claim 23, wherein the gel precursor cluster includes the first polymer unit having one or more nucleophilic functional groups in a side chain or at an end and the second polymer unit having one or more electrophilic functional groups in a side chain or at an end, and the gel precursor cluster stores the following two types of polymer compositions (a) and (b) without mixing those polymer compositions:
   (a) a polymer composition including a first gel precursor cluster that has a content of the first polymer unit higher than a content of the second polymer unit; and
   (b) a polymer composition including a second gel precursor cluster that has a content of the second polymer unit higher than a content of the first polymer unit.

### Advantageous Effects of Invention

According to an embodiment of the present invention, there is provided a gel material for ophthalmic treatment which has a low swelling pressure, an appropriate elastic force, and no toxicity to ocular tissues, specifically to retinas, and which is capable of stably maintaining a long-term stable tamponade effect. The gel material herein is a non-swelling material which does not cause undesirable swelling even after intraocular injection and after a long time passage, and which has excellent biocompatibility and biodegradability. Therefore, the gel material has an effect of not causing clouding or inflammation due to intraocular injection and can be used as a synthetic vitreous body which has not been put to practical use so far. Furthermore, the gel material has hydrophilicity with a high moisture content exceeding 90%, so that the gel material has permeability to substances such as water, ions, nutrients, and chemical mediators through the vitreous body.

Still further, a few minutes are required in gelation time in which gel precursor clusters crosslink to form a hydrogel, so that the gel precursor clusters can be intraocularly injected in a solution state and allowed to gel in vivo so as to be self-assembled. In this manner, since the gel material can be injected as a solution that contains the gel precursor clusters in a sol state before gelation, the gel material has excellent operability and can be used in a needle thinner than a typically-used injection needle (25 gauge), which leads to an advantage that a load applied to an eyeball and the time required for treatment can be reduced. Unlike gas tamponade materials and liquid tamponade materials such as silicone oil, the gel material does not require a patient to keep lying prone after surgery or does not require removal of the materials after surgery, so that it is possible to reduce the burden on the patient.

### Brief Description of Drawings

Fig. 1 is a schematic view illustrating a structure and a manufacturing process of a hydrogel in a gel material for ophthalmic treatment of the present invention.
Fig. 2 is a graph illustrating temporal change of elastic modulus in a typical gelation process.
Fig. 3 is a graph illustrating gelation time in regard to Comparative Example (○) and the present invention (Δ) in which a gel precursor cluster 1 [TAPEG + TNPEG] is used.
Fig. 4 is a graph illustrating gelation time in regard to Comparative Example (□) and the present invention (○) in which a gel precursor cluster 2 [SHPEG + MAPEG] is used.
Fig. 5 is a graph illustrating a size distribution of the gel precursor cluster 1 [TAPEG + TNPEG].
Fig. 6 is a graph illustrating measurement results of dynamic viscosity characteristics of the gel precursor cluster 1 [TAPEG + TNPEG] at a gelation critical point.
Fig. 7 is a graph illustrating a fractal dimension of the gel precursor cluster 1 [TAPEG + TNPEG].
Fig. 8 is a graph illustrating polymer concentration dependency of elastic modulus in a hydrogel 1 [TAPEG + TNPEG].
Fig. 9 is a graph illustrating polymer concentration dependency of elastic modulus in a hydrogel 2 [SHPEG + MAPEG].
Fig. 10 is a graph illustrating measurement results of temporal change of swelling pressure in regard to the hydrogel 2 [SHPEG + MAPEG].
Fig. 11 is a graph illustrating measurement results of intraocular pressure before surgery, 3 days, 7 days, and 28 days after surgery when the hydrogel 2 [SHPEG + MAPEG] is intraocularly injected into a rabbit with the vitreous body resected.
Fig. 12 is an image of the anterior eye of the rabbit injected with the hydrogel 2 [SHPEG + MAPEG].
Fig. 13 is an image of the eyeground of the rabbit injected with the hydrogel 2 [SHPEG + MAPEG].

### Description of Embodiments

Hereinafter, an embodiment of the present invention will be described. The scope of the present invention is not limited by these descriptions, and those other than the following illustration can be appropriately modified and implemented without departing from the gist of the present invention.

A gel material for ophthalmic treatment of the present invention includes a hydrogel in which gel precursor clusters crosslink to form a three-dimensional network. The gel material for ophthalmic treatment has characteristics such as a low swelling pressure, an appropriate elastic force, and what is more, a non-swelling characteristic, no toxicity, and biocompatibility, so that the gel material is preferably used as a replacement material for the vitreous body (what is called an intraocular tamponade material) in ophthalmic surgical operations such as vitreous surgeries, and due to its outstanding characteristics, the gel material can be used as a synthetic vitreous body. Furthermore, the gel material gels in a short time so that, in applying the gel material intraocularly, for example, as described later, the gel material can be injected as a solution containing the gel precursor clusters and allowed to gel in vivo.

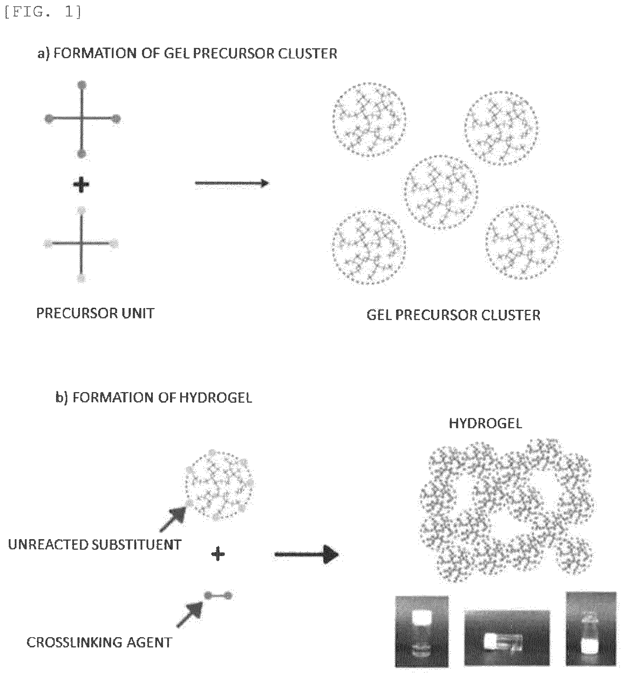

Fig. 1 is a schematic view illustrating a structure and a manufacturing process of the hydrogel in the gel material for ophthalmic treatment of the present invention. In a first step, as illustrated in Fig. 1a), monomer units or polymer units (hereinafter referred to as "precursor units") which ultimately form the hydrogel are reacted to each other in a state on the verge of gelation so as to form polymer clusters having a structure in a pre-gel state, that is, in a sol state. In a subsequent second step, as illustrated in Fig. 1b), an appropriate crosslinking agent is added to the clusters, and the clusters (gel precursor clusters) are further reacted to each other and allowed to crosslink three dimensionally so as to yield the hydrogel as an end product. Herein, the gel precursor clusters are not necessarily limited to a single variety having the same composition as described below, but a plurality of gel precursor clusters having different compositions may also be used.

In the aforementioned method, the gel precursor clusters are used as what is called precursors or intermediates of a final gel, so that it is possible to form the gel in a short time even in low-concentration polymer content and to control an elastic modulus and a swelling degree of the gel even in a region with low elasticity. Herein, the "gel" generally refers to a dispersion having high viscosity and losing fluidity.

### (1) Gel Precursor Cluster

The gel precursor cluster used in the gel material for ophthalmic treatment of the present invention is a sol polymer cluster obtained by reacting or crosslinking the precursor units in a state on the verge of gelation as described above, that is, in concentration less than a critical gelation concentration. Herein, the "critical gelation concentration" is also referred to as the lowest concentration of gelation, representing the minimum concentration of the precursor units required for achieving gelation in a system that forms a gel having a three-dimensional structure by crosslinking of specific precursor units. In the present invention, for example, in a system including two or more types of precursor units, the term "critical gelation concentration" includes, not only a case where concentrations of those precursor units fail to reach the concentration that reaches gelation, but also a case where a concentration of one precursor unit is low, that is, a case where gelation does not occur due to a non-equivalent ratio of the precursor units.

Although the gel precursor cluster has a structure with the mutually bonded or crosslinked precursor units, the gel precursor cluster is formed in a pre-gel state so that the precursor units include unreacted substituents. Crosslinks of the substituents in the reaction between the gel precursor clusters form the final gel.

The gel precursor cluster has a relationship of G' < G" where G' represents a storage elastic modulus and G" represents a loss elastic modulus. Generally, in a pre-gel polymer, it is known that a value of loss elastic modulus G" is larger than that of storage elastic modulus G', and as gelation proceeds, the values of these physical properties reverse, and G" becomes larger. The point where G' = G" is what is called a gelation point. Therefore, the gel precursor cluster being G' < G" indicates that the gel precursor cluster is in a sol state before gelation. Preferably, G' < G" < 100 G' at a frequency of 1 Hz.

Preferably, G" of the gel precursor cluster is in the range of 0.005 to 5 Pa at the frequency of 1 Hz, more preferably in the range of 0.01 to 1 Pa, and still more preferably in the range of 0.01 to 0.5 Pa. These elastic moduli may be calculated by a known method such as dynamic viscoelasticity measurement with a known measuring device such as a rheometer.

The gel precursor cluster in the present invention preferably has a fractal dimension of 1.5 to 2.5. More preferably, the gel precursor cluster has a fractal dimension of 1.5 to 2.0. Herein, the fractal dimension is an index that shows how close to a three-dimensional structure the crosslinked structure formed by the polymer units is. In regard to a calculation method of the fractal dimension, refer to, for example, (W. Hess, T. A Vilgis, and H. H Winter, Macromolecules 21, 2536 (1988)). Specifically, the fractal dimension may be calculated by the dynamic scaling theory based on, for example, changes in dynamic viscoelastic characteristics at the gelation point.

The gel precursor cluster in the present invention preferably has a diameter of 10 to 1000 nm, and more preferably 50 to 200 nm. In addition, it is preferable that the gel precursor cluster having a diameter of about 100 nm accounts for the greatest proportion in the distribution.

In regard to the precursor units used to form a gel precursor cluster, any precursor units known in the related art may be used as long as they are monomers or polymers capable of forming a gel by a gelation reaction (a crosslinking reaction or the like) in a solution, depending on the application and shape of the final gel. More specifically, in the final gel obtained from the gel precursor cluster, it is preferable to use polymer units, as the precursor units, capable of forming a network, particularly, a three-dimensional network by crosslinks of polymers.

Examples of such monomer units include molecules having a vinyl skeleton. Typical examples of the polymer units include polymer species with a plurality of branches having a polyethylene glycol skeleton. Particularly, polymer species with four branches having a polyethylene glycol skeleton are preferable. A gel formed by such a tetra-branched polyethylene glycol skeleton is generally known as a Tetra-PEG gel in which a network is formed by an AB-type cross-end coupling reaction between two types of tetra-branched polymers each having an electrophilic functional group such as an active ester structure and a nucleophilic functional group such as an amino group at an end. It has been reported that a Tetra-PEG gel has an ideal homogeneous network without heterogeneity in a polymer network in a size region of 200 nm or less (Matsunaga et al., Macromolecules, Vol. 42, No. 4, pp. 1344-1351, 2009). It is possible to prepare a Tetra-PEG gel easily and instantly by simply mixing two solutions each of which is a polymer solution, and it is possible to control gelation time by adjusting the pH or ionic strength of the Tetra-PEG gel at the time of gel preparation. Since this gel contains PEG as a main component, it has excellent biocompatibility.

It should be noted that polymers other than one having a polyethylene glycol skeleton may also be used as long as they crosslink to form a network. For example, a polymer having a polyvinyl skeleton such as methyl methacrylate may also be used.

Although formation of the polymer units is not necessarily limited to the following means, in order to form a network in the final gel, it is preferable to react and crosslink two types of polymer species: a first polymer having one or more nucleophilic functional groups in a side chain or at an end; and a second polymer having one or more electrophilic functional groups in a side chain or at an end. Herein, the total number of the nucleophilic functional groups and the electrophilic functional groups is preferably 5 or more. It is further preferred that these functional groups are present at the ends. Furthermore, the gel precursor cluster may have a content of first polymer units higher than that of second polymer units or may have the content of the second polymer units higher than that of the first polymer units. In a preferred aspect as described below, two or more types of gel precursor clusters having different compositions, as described above, crosslink to yield a hydrogel.

Examples of the nucleophilic functional groups in the polymer units include amino groups, -SH, or -CO₂PhNO₂ (Ph represents o-, m-, or p-phenylene groups), and those skilled in the art may appropriately use any known nucleophilic functional groups. Preferably, the nucleophilic functional groups are -SH groups. The nucleophilic functional groups may be the same or different but preferably the same. The same functional groups lead to homogeneous reactivity with the electrophilic functional groups that form crosslinks together, which makes it easy to obtain a gel having a homogeneous three-dimensional structure.

As the electrophilic functional groups in the polymer units, active ester groups may be used. Examples of such active ester groups include N-hydroxy-succinimidyl (NHS) groups, sulfosuccinimidyl groups, maleimidyl groups, phthalimidyl groups, imidazoyl groups, acryloyl groups, or nitrophenyl groups, and those skilled in the art may appropriately use any known active ester groups. Preferably, the electrophilic functional groups are maleimidyl groups. The electrophilic functional groups may be the same or different but preferably the same. The same functional groups lead to homogeneous reactivity with the nucleophilic functional groups that form crosslinks together, which makes it easy to obtain a gel having a homogeneous three-dimensional structure.

A combination of -SH groups and maleimidyl groups is a preferred combination of the nucleophilic functional groups and the electrophilic functional groups in the polymer units included in the gel material for ophthalmic treatment of the present invention. Such a combination is preferable from a viewpoint that an inflammatory reaction may be minimized when the gel material is intraocularly applied as a tamponade material or a synthetic vitreous body.

A preferred specific example of the polymer units having the nucleophilic functional groups at the end includes a compound with four branches having a polyethylene glycol skeleton and having an amino group at an end, which is represented by the following Formula (I) but is not limited thereto.

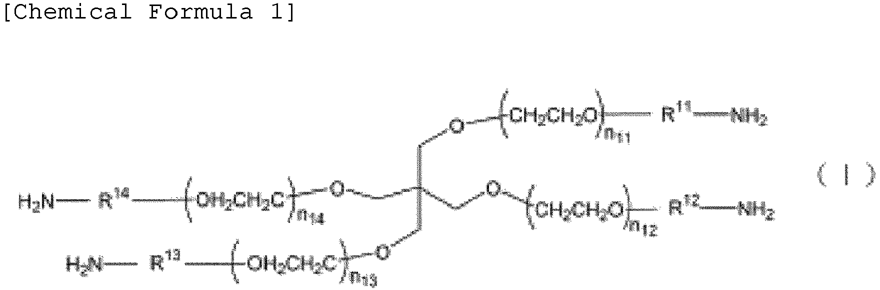

In Formula (I), R¹¹ to R¹⁴, the same or different, represent a C₁-C₇ alkylene group, a C₂-C₇ alkenylene group, -NH-R¹⁵-, -CO-R¹⁵-, -R¹⁶-O-R¹⁷-, -R¹⁶-NH-R¹⁷-, R¹⁶⁻CO₂-R¹⁷, - R¹⁶-CO₂-NH-R¹⁷ -R¹⁶⁻CO-R¹⁷-, or -R¹⁶-CO-NH-R¹⁷ -, where R¹⁵ represents a C₁-C₇ alkylene group, R¹⁶ represents a C₁-C₃ alkylene group, and R¹⁷ represents a C₁-C₅ alkylene group.)

The numbers n₁₁ to n₁₄ may be the same or different. As the values of n₁₁ to n₁₄ get closer to each other, a homogeneous three-dimensional structure can be obtained, which leads to high intensity. Therefore, in order to obtain a high-intensity gel, the numbers n₁₁ to n₁₄ are preferably the same. Extremely high values of n₁₁ to n₁₄ reduce the strength of gel, and extremely low values of n₁₁ to n₁₄ make it difficult to form a gel due to steric hindrance of the compound. Therefore, each of n₁₁ to n₁₄ is an integer of 25 to 250, preferably 35 to 180, more preferably 50 to 115, and still more preferably 50 to 60. A molecular weight of the compound is 5 × 10³ to 5 × 10⁴ Da, preferably 7.5 × 10³ to 3 × 10⁴ Da, and more preferably 1 × 10⁴ to 2 × 10⁴ Da.

In the above Formula (I), R¹¹ to R¹⁴ are linker moieties that link a functional group and a core moiety. R¹¹ to R¹⁴ may be the same or different but preferably the same in order to produce a high-intensity gel having a homogeneous three-dimensional structure. R¹¹ to R¹⁴ represent a C₁-C₇ alkylene group, a C₂-C₇ alkenylene group, -NH-R¹⁵-, -CO-R¹⁵-, -R¹⁶-O-R¹⁷-, -R¹⁶-NH-R¹⁷-, -R¹⁶-CO₂-R¹⁷-, - R¹⁶-CO₂-NH-R¹⁷-, -R¹⁶-CO-R¹⁷-, or -R¹⁶-CO-NH-R¹⁷-. Herein, R¹⁵ represents a C₁-C₇ alkylene group. R¹⁶ represents a C₁-C₃ alkylene group. R¹⁷ represents a C₁-C₅ alkylene group.

Herein, the "C₁-C₇ alkylene group" indicates an alkylene group having one to seven carbon atoms which may have a branch, or a linear C₁-C₇ alkylene group or a C₂-C₇ alkylene group having one or at least two branches (the number of carbon atoms is two to seven, including branches). Examples of the C₁-C₇ alkylene group include a methylene group, an ethylene group, a propylene group, and a butylene group. Examples of the C₁-C₇ alkylene group include -CH₂-, -(CH₂)₂-, -(CH₂)₃-, -CH(CH₃)-, -(CH₂)₃-, -(CH(CH₃))₂, -(CH₂)₂-CH (CH₃)-, -(CH₂)₃-CH(CH₃)-, -(CH₂)₂-CH(C₂H₅)-, -(CH₂)₆-,-(CH₂)₂-C(C₂H₅)₂-, and- (CH₂)₃C(CH₃)₂CH₂-.

The "C₂-C₇ alkenylene group" is a group having a chain with one or at least two double bonds or a branched-chain alkenylene group with two to seven carbon atoms, example of which includes a divalent group having a double bond obtained by eliminating two to five hydrogen atoms of adjacent carbon atoms from the alkylene group.

In an aspect in which -SH groups are used as the nucleophilic functional groups, as described above, it is possible to use a compound having a structure in which the -SH groups are introduced instead of -NH₂ groups at the ends of the four branched chains having the polyethylene glycol skeleton in Formula (I).

On the other hand, a preferred specific example of the polymer units having the electrophilic functional groups at the end includes a compound with four branches having a polyethylene glycol skeleton and having an N-hydroxy-succinimidyl (NHS) group at an end, which is represented by the following Formula (II) but is not limited thereto.

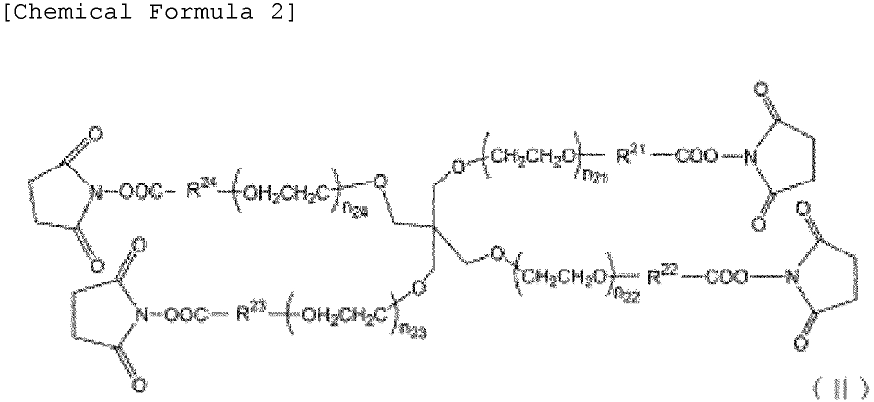

In the above Formula (II), n₂₁ to n₂₄ may be the same or different. As the values of n₂₁ to n₂₄ get closer to each other, a homogeneous three-dimensional structure can be obtained in a gel, leading to high intensity, so that n₂₁ to n₂₄ are preferably the same. Extremely high values of n₂₁ to n₂₄ reduce the strength of gel, and extremely low values of n₂₁ to n₂₄ make it difficult to form a gel due to steric hindrance of the compound. Therefore, each of n₂₁ to n₂₄ is an integer of 5 to 300, preferably 20 to 250, more preferably 30 to 180, still more preferably 45 to 115, and still more preferably 45 to 55. A molecular weight of the second tetra-branched-compound of the present invention is 5 × 10³ to 5 × 10⁴ Da, preferably 7.5 × 10³ to 3 × 10⁴ Da, and more preferably 1 × 10⁴ to 2 × 10⁴ Da.

In the above Formula (II), R²¹ to R²⁴ are linker moieties that link a functional group and a core moiety. R²¹ to R²⁴ may be the same or different, but in order to produce a high-intensity gel having a homogeneous three-dimensional structure, R²¹ to R²⁴ are preferably the same. In Formula (II), R²¹ to R²⁴, the same or different, represent a C₁-C₇ alkylene group, a C₂-C₇ alkenylene group, -NH-R²⁵-, -CO-R²⁵-, -R²⁶-O-R²⁷-, -R²⁶-NH-R²⁷-, -R²⁶-CO₂-R²⁷-,-R²⁶-CO₂-NH-R¹⁷-, -R²⁶-CO-R²⁷-, or -R²⁶-CO-NH-R²⁷-. Herein, R²⁵ represents a C₁-C₇ alkylene group. R²⁶ represents a C₁-C₃ alkylene group. R²⁷ represents a C₁-C₅ alkylene group.

In an aspect in which maleimidyl groups are used as the electrophilic functional groups, as described above, it is possible to use a compound having a structure in which the maleimidyl groups are introduced instead of NHS groups at the ends of the four branched chains having the polyethylene glycol skeleton in Formula (I).

Herein, the alkylene group and the alkenylene group may have one or more optional substituents. Examples of the substituents include alkoxy groups, halogen atoms (which may be any one of a fluorine atom, a chlorine atom, a bromine atom, or an iodine atom), amino groups, mono- or di-substituted amino groups, substituted silyl groups, acyl groups, and aryl groups, but the substituents are not limited thereto. When an alkyl group has two or more substituents, those substituents may be the same or different. Similarly, alkyl moieties of other substituents including the alkyl moieties (for example, an alkyloxy group, and an aralkyl group) may be the same or different.

Furthermore, herein, when a certain functional group is defined that it "may have a substituent(s)", types of the substituent(s), substitution position(s), and the number of the substituents are not particularly limited, and when a certain functional group has two or more substituents, those substituents may be the same or different. Examples of the substituents include alkyl groups, alkoxy groups, hydroxyl groups, carboxyl groups, halogen atoms, sulfo groups, amino groups, alkoxycarbonyl groups, and oxo groups, but the substituents are not limited thereto. These substituents may further include a substituent.

In the polymer units of Formulae (I) and (II), it is possible to obtain a gel precursor cluster having a structure in which these polymer units bond by amide bonds. As described later, in that case, even in the final gel, each polymer unit has a structure crosslinked by the amide bonds.

### (2) Hydrogel in Gel Material for Ophthalmic Treatment of the Present Invention

The hydrogel, the main component of the gel material for ophthalmic treatment of the present invention, maintains a low swelling pressure stable for a long period of time and an appropriate elastic modulus while having a low-concentration polymer content, and is suitable as an intraocular tamponade material and a synthetic vitreous body in ophthalmic surgical operations such as vitreous surgeries.

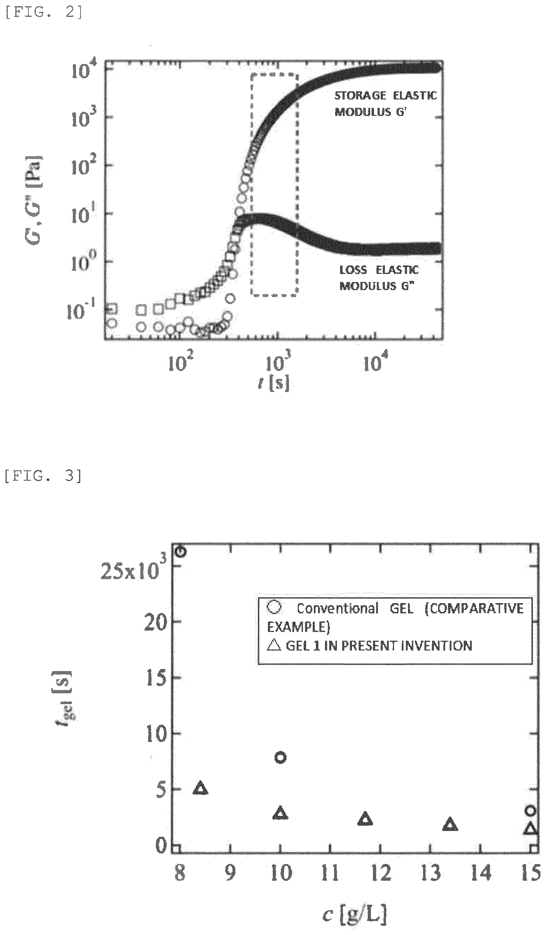

As illustrated in Fig. 2, since the elastic modulus about the gelation point generally increases drastically, it is difficult to obtain a gel having a low elastic modulus controlled to a specific value in a range with low elastic modulus such as 10 to 1000 Pa. In contrast, the gel in the present invention is prepared through the aforementioned gel precursor cluster, so that the gel has an elastic modulus controlled in the region with low elasticity.

Accordingly, the hydrogel herein includes the polymer units that crosslink to form a three-dimensional network and has a low-concentration polymer content, a low elastic modulus in the low region, and a specific fractal dimension.

From a viewpoint that the hydrogel in the gel material for ophthalmic treatment of the present invention is used as an intraocular tamponade material and a synthetic vitreous body in ophthalmic surgical operations, it is desirable that the hydrogel has the following physical properties.

The polymer content in the hydrogel of the present invention is 50 g/L or less, preferably 40 g/L or less, and more preferably 15 to 30 g/L.

The hydrogel of the present invention has a storage elastic modulus G' of 1 to 10000 Pa, and preferably 10 to 1000 Pa. This range corresponds to the vitreous body (several tens Pa) in a living body, and when the gel is intraocularly used as a replacement for the vitreous body, the above range is preferable in order to press retinas and to prevent the retinas from being detached. Furthermore, the hydrogel of the present invention preferably has a loss elastic modulus G" of 1 to 100 Pa. These elastic moduli may be calculated by a known method with a known measuring device.

Still further, the hydrogel of the present invention preferably has a fractal dimension of 1.5 to 2.5. More preferably, the hydrogel has a fractal dimension of 1.5 to 2.0. The fractal dimension is an index indicating how close to a three-dimensional structure the crosslinked structure in the gel is, and a calculation method of the fractal dimension is known in the technical field as described above.

In an aqueous solution, the hydrogel of the present invention has swelling pressure of 0.1 to 5 kPa and a swelling degree in a range where the volume of the hydrogel in a temperature of 30 to 40°C changes from 90 to 500% of the volume at the time of gel formation. A low swelling pressure indicates that the pressure applied to the outside, when the gel is placed in a closed space, is low. In other words, when using the gel intraocularly, it is possible to prevent an increase in intraocular pressure caused by moisture absorption and swelling of the gel over time, leading to a disadvantage such that undesirable cataracts and the like develop after surgery.

In regard to the polymer units included in the hydrogel of the present invention, those similar to the aforementioned gel precursor clusters may be used. In a preferred aspect, when a gel precursor cluster includes first polymer units having one or more nucleophilic functional groups in a side chain or at an end and second polymers unit having one or more electrophilic functional group in a side chain or at an end, the gel precursor cluster may include two types of gel precursor clusters: a first gel precursor cluster having a composition in which a content of the first polymer units is higher than that of the second polymer units; and a second gel precursor cluster having a composition in which the content of the second polymer units is higher than that of the first polymer units, and it is possible to form a hydrogel having a three-dimensional network in which these two types of gel precursor clusters having different compositions are crosslinked to each other.

### (3) Method for Producing Gel Material for Ophthalmic Treatment

Typically, the gel material for ophthalmic treatment of the present invention may be manufactured by the following gelation reaction process.
a) a step of crosslinking monomer units or polymer units (precursor units) at a concentration less than a critical gelation concentration so as to form gel precursor clusters (Fig. 1a)
b) a step of crosslinking the gel precursor clusters with each other to obtain a gel having a three-dimensional network which is a final target substance (Fig. 1b)

In Step a), as described above, by adjusting the initial concentration of the precursor units, the precursor units are reacted at the concentration less than the critical gelation concentration to obtain polymer clusters having a structure in a sol state before gelation, preferably, in a state on the verge of gelation. Since the clusters may be expressed as what is called precursors of a final gel, the clusters herein are referred to as the "gel precursor clusters".

As a method for adjusting the initial concentration of the precursor units to a condition lower than the critical gelation concentration, for example, when using two types of polymer units having a nucleophilic functional group(s) or an electrophilic functional group(s) as described above, the following conditions may be used: making those polymer units to have equivalent but insufficient amounts for overall gelation at low concentrations; or making one polymer unit at low concentration, that is, making those polymer units to have non-equivalent amounts so as not to reach gelation.

Generally, a critical gelation concentration (minimum gelation concentration) depends on types of precursor units used, but the concentration is known in the technical field or is readily experimentally comprehensible by those skilled in the art. A typical critical gelation concentration is 5 to 50g/L, and the lower limit is a concentration of about 1/5 of an overlap concentration. Herein, the overlap concentration is a concentration at which the polymer units fill a solution. In regard to a calculation method of the overlap concentration, refer to, for example, Polymer Physics (M. Rubinstein, R. Colby). Specifically, for example, the overlap concentration may be determined by measuring viscosity of a dilute solution, using the Flory-Fox equation.

Step a) can be typically performed by mixing or stimulating solutions containing two types of precursor units. Step a) may also be performed by radical polymerization of monomers with a radical initiator. A concentration, an addition rate, a mixing rate, and a mixing ratio of each solution are not particularly limited, and those skilled in the art may appropriately adjust those conditions. Even in using three or more precursor units, it is clear that solutions containing corresponding numbers of precursor units can be prepared in a similar manner and mixed in an appropriate manner. When a solution containing precursor units is an aqueous solution, it is possible to use an appropriate pH buffer solution such as a phosphate buffer solution.

In regard to a mixing means, it is possible to use, for example, a syringe containing two solutions mixed as recited in International Publication WO 2007/083522. Temperature of the two solutions at the time of mixing is not particularly limited and may be any temperature as long as each precursor unit is dissolved, and each solution has fluidity. For example, the temperature of the solutions at the time of mixing may be in the range of 1°C to 100°C. The temperature of the two solutions may be different but is preferably the same so that the two solutions are easily mixed.

Next, in Step b), the gel precursor clusters obtained in Step a) are further reacted and allowed to crosslink three-dimensionally with each other so as to obtain a hydrogel which is an end product. As described above, since the gel precursor clusters are formed in a state before reaching the gelation point, a substituent used for crosslinking in each precursor unit remains unreacted. The substituent in one gel precursor cluster is reacted with a remaining substituent of another gel precursor cluster, and those substituents are allowed to crosslink with each other, thereby forming the final gel.

Preferably, in this process, a crosslinking agent to make the gel precursor clusters crosslink with each other may be added or the gel precursor clusters may be stimulated. In regard to such a crosslinking agent, one having the same substituent as the crosslinking group in each polymer unit may be used, or the polymer unit itself may be used as a crosslinking agent and additionally applied to the gel precursor clusters. Typically, as such a crosslinking agent, bis-(sulfosuccinimidyl) glutarate (BS₂G), DL-dithiothreitol (DTT), a synthetic peptide having a thiol group at an end, or the like may be used.

For example, in Step a), when two types of polymer units having the nucleophilic functional group(s) or the electrophilic functional group(s) are reacted in a non-equivalent amount to obtain the gel precursor clusters, it is possible to crosslink the gel precursor clusters with each other by adding a crosslinking agent having a functional group at lower concentration. As such a stimulus, for example, the functional group(s) (such as a maleimide group) that causes photodimerization may be irradiated with ultraviolet light.

In Step b), it is possible to obtain the final gel a reaction time within 2 hours, and preferably within 1 hour. Generally, when preparing a gel containing low-concentration polymers, a long reaction time is required (for example, about 8 hours when a polymer content is 10 g/L or less, though the reaction time depends on a system). On the contrary, in production of the hydrogel according to the present invention that contains the gel precursor clusters, it is possible to prepare the gel in a much shorter time.

Other conditions and the like of the reaction solution in Step b) are similar to those in Step a).

### (4) Usage of Gel Material for Ophthalmic Treatment

The gel material for ophthalmic treatment of the present invention can be injected intraocularly by any suitable means. Preferably, an aqueous solution or the like containing the gel precursor clusters is injected intraocularly with a syringe and directly gels intraocularly, or in vivo, to form a hydrogel. Since the gel material can be injected as a solution that contains the gel precursor clusters in a sol state before gelation, the gel material has excellent operability and can be used in a needle thinner than a typically-used injection needle (25 gauge). The factor for making such a technique feasible is that, as described above, the hydrogel used in the present invention can gel in a much shorter time than one in the related art.

Accordingly, the present invention also relates to a polymer composition for ophthalmic treatment containing the gel precursor clusters which is to be intraocularly injected when forming the hydrogel in vivo in this manner, and also relates to a kit including the composition. A preferable aspect of the polymer composition for ophthalmic treatment is an aqueous solution containing the gel precursor clusters.

The polymer composition for ophthalmic treatment containing the gel precursor clusters preferably has the pH which is adjusted to a physiological condition (about pH 7.4) and may be adjusted with any pH adjusting agents or buffers and the like. In addition, in order to make conditions in the solution similar to those in the aqueous humor, ionic salts such as sodium chloride and magnesium chloride can be included in the solution.

As described above, when the hydrogel is formed by crosslinks of two or more gel precursor clusters having different compositions, before the polymer composition for ophthalmic treatment is intraocularly injected, such plural gel precursor clusters should not be mixed and should be stored as separate and independent polymer compositions. Then, at the time of intraocular injection, these plural polymer compositions may be mixed and intraocularly injected with a syringe. When a crosslinking agent is used for hydrogel formation, the crosslinking agent may be contained in any one of these plural polymer compositions, or a separate solution containing only the crosslinking agent may be mixed before injection.

More specifically, the kit including the polymer composition for ophthalmic treatment may store the following two types of polymer compositions (a) and (b) without mixing those polymer compositions with each other.
(a) a polymer composition containing the first gel precursor cluster which has the content of the first polymer units higher than the content of the second polymer units;
(b) a polymer composition containing the second gel precursor cluster which has the content of the second polymer units higher than the content of the first polymer units

Furthermore, as mentioned above, the kit may further include a crosslinking agent.

### Examples

Hereinafter, the present invention will be described in more detail with reference to Examples, but the present invention is not limited by these Examples.

### Example 1

### Synthesis of Polymer Units

Tetrahydroxyl-polyethylene glycol (THPEG) having a hydroxyl group at an end was aminated and succinimidylated to obtain tetraamine-polyethylene glycol (TAPEG) and tetra N-hydroxy-succinimidyl-polyethylene glycol (NHS-PEG) (TNPEG).

In regard to tetrathiol-polyethylene glycol (SHPEG) having a -SH group at an end and tetramaleimidyl-polyethylene glycol (MAPEG) having a maleimidyl group at an end, those commercially available from NOF Corporation were used. Each compound has a molecular weight of 100,000.

In the following test, ¹H NMR spectrum was analyzed with JNM-ECS 400 (400 MHz) manufactured by JEOL. Deuterated chloroform was used as a solvent, and tetramethylsilane was used as an internal standard. A molecular weight was determined with Bruker Daltonics mass spectrometer Ultraflex III in a linear positive ion mode.

### 1. Synthesis of THPEG:

An initiator, pentaerythritol (0.4572 mmol, 62.3 mg), was dissolved in 50 mL of a mixed solvent of DMSO/THF (v/v = 3 : 2). Using potassium naphthalene (0.4157 mmol, 1.24 mg) as a metalizing agent, ethylene oxide (200 mmol, 10.0 mL) was added to the solvent, and the mixture was heated and stirred at 60°C under an Ar atmosphere for about 2 days. After the completion of the reaction, the resultant was reprecipitated in diethyl ether and filtered so as to take out precipitates. In addition, the precipitates were washed three times with diethyl ether to obtain a white solid, and the white solid was dried under reduced pressure to yield 20k of THPEG.

### 2. Synthesis of TAPEG:

THPEG (0.1935 mmol, 3.87g, 1.0 equiv) was dissolved in benzene and lyophilized, and then dissolved in 62 mL of THF to which triethylamine (TEA) (0.1935 mmol, 3.87g, 1.0 equiv) was added. To another recovery flask, 31 mL of THF and methanesulfonyl chloride (MsCl) (0.1935 mmol, 3.87g, 1.0 equiv) were added, and the mixture was cooled on ice. The MsCl-containing THF solution was added to the THPEG and TEA-containing THF solution by drops for about 1 minute, and the mixture was stirred on ice for 30 minutes and then stirred at room temperature for 1.5 hours. After the completion of the reaction, the resultant was reprecipitated in diethyl ether and filtered so as to take out precipitates. In addition, the precipitates were washed three times with diethyl ether to obtain a while solid, and the white solid was transferred to a recovery flask to which 250 mL of 25% aqueous ammonia was added, and the mixture was stirred for 4 days. After the completion of the reaction, the solvent was distilled by an evaporator under reduced pressure, and water was dialyzed in an external solution a couple of times and lyophilized to yield white solid TAPEG. The chemical formula of the prepared TAPEG is illustrated in Formula (Ia). In Formula (Ia), n₁₁ to n₁₄ are 50 to 60 when a molecular weight of TAPEG is about 10,000 (10 kDa) and are 100 to 115 when the molecular weight is about 20,000 (20 kDa).

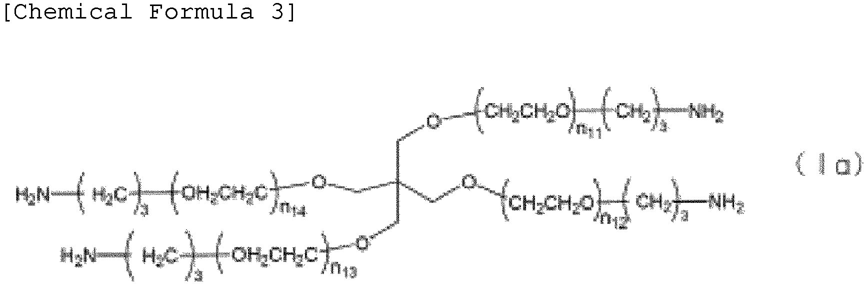

### 3. Synthesis of TNPEG:

THPEG (0.2395 mmol, 4.79g, 1.0 equiv) was dissolved in THF to which 0.7 mol/L glutaric acid/THF solution (4.790 mmol, 6.85 mL, 20 equiv) was added, and the mixture was stirred under an Ar atmosphere for 6 hours. After the completion of the reaction, the resultant was added to 2-propanol by drops, and the mixture was centrifuged three times. The white solid obtained was transferred to a 300 mL recovery flask, and the solvent was distilled by the evaporator under reduced pressure. A residue was dissolved in benzene, and an insoluble matter was removed by filtration. The filtrate obtained was lyophilized to remove the solvent, thereby yielding white solid Tetra-PEG-COOH having an end modified by a carboxyl group. This Tetra-PEG-COOH (0.2165 mmol, 4.33g, 1.0 equiv) was dissolved in THF to which N-hydrosuccinamide (2.589 mmol, 0.299g, 12 equiv) and N, N'-diisopropylsuccinamide (1.732 mmol, 0.269 mL, 8.0 equiv) were added, and the mixture was heated and stirred at 40°C for 3 hours. After the completion of the reaction, the solvent was distilled by the evaporator under reduced pressure. The solvent was dissolved in chloroform and extracted three times with saturated saline so as to take out a chloroform layer. In addition, after dehydration and filtration with magnesium sulfate, the solvent was evaporated by the evaporator under reduced pressure. The residue obtained was lyophilized with benzene to yield white solid TNPEG. The chemical formula of the TNPEG prepared is illustrated in Formula (IIa). In Formula (IIa), n₂₁ to n₂₄ are 45 to 55 when a molecular weight of TNPEG is about 10,000 (10k) and are 90 to 115 when the molecular weight is about 20,000 (20k).

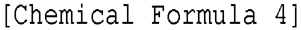

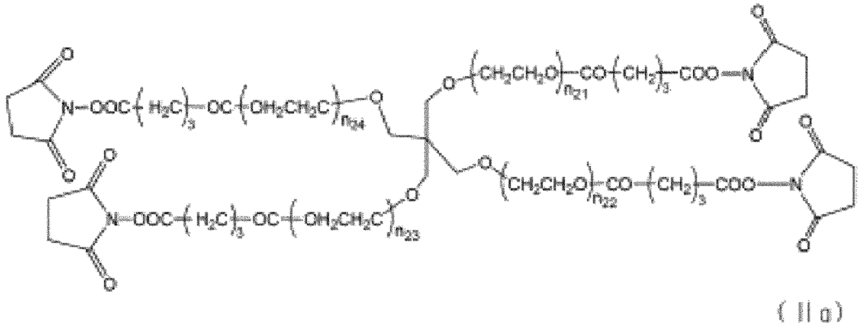

### Example 2

### Synthesis of Gel Precursor Clusters

Gel precursor clusters, which are to be precursors in a gelation reaction, were synthesized in the following manner.

### (1) Gel Precursor Cluster 1 [TAPEG + TNPEG]

First, TAPEG (1.0 x 10⁴ g/mol) and TNPEG (1.0 x 10⁴ g/mol) synthesized in Example 1 were dissolved in an equivalent amount of 81 mM phosphate buffer and citrate-phosphate buffer, respectively. At this time, a mole ratio was TAPEG/TNPEG = 1/0.23, and the total polymer concentration was 60 g/L. The two solutions obtained were mixed in another container and defoamed and stirred by a rotation-revolution mixer. The mixed solution was then quickly transferred to a Falcon tube and capped to prevent dryness, and the mixed solution was allowed to stand at room temperature for 12 hours.

### (2) Gel Precursor Cluster 2 [SHPEG + MAPEG]

Using SHPEG and MAPEG, gel precursor clusters 2 were synthesized in a similar manner. The total polymer concentration was 60 g/L. At this time, prepared was a plurality of samples containing two types of gel precursor clusters in which either SHPEG or MAPEG is included excessively so that SHPEG : MAPEG became equivalent to a mole ratio of (1 - r) : r.

### Example 3

### Synthesis of Hydrogel

Using the gel precursor clusters synthesized in Example 2, a hydrogel was synthesized in the following manner.

### 1) Hydrogel 1 [TAPEG + TNPEG]

A solution of the gel precursor clusters 1 obtained in Example 2 was diluted with water so as to be 25 g/L. An amount of unreacted amino groups in the solution was calculated, and an equivalent amount of a crosslinking agent (Bis-(sulfosuccinimidyl) glutarate (BS₂G)) was added to the solution, and the mixture was defoamed and stirred with a rotation-revolution mixer. The mixed solution was then quickly transferred to a Falcon tube and capped to prevent dryness, and the mixed solution was allowed to stand at room temperature for 12 hours.

Fig. 3 illustrates reaction time when gelation was performed by changing concentrations of the gel precursor clusters. In Fig. 3, the gelation time t_{gel} (second) is taken along the ordinate, and the polymer content c (g/L) in the hydrogel is taken along the abscissa. In the drawing, Δ represents Example in which the hydrogel of the present invention gelled from the gel precursor clusters, and ○ represents Comparative Example in which a hydrogel gelled directly from polymer units by a conventional method without using gel precursor clusters. The result shows that it is possible to obtain a hydrogel with a short reaction time when gelation is performed from gel precursor clusters. Particularly, at a concentration in polymer content as low as about 8 g/L, the conventional method required 7 hours or more of gelation time, whereas the gel precursor clusters of the present invention gelled within 1.5 hours. When using the gel precursor clusters in a region with a higher concentration, the gelation time was less than 30 minutes.

### 2) Hydrogel 2 [SHPEG + MAPEG]

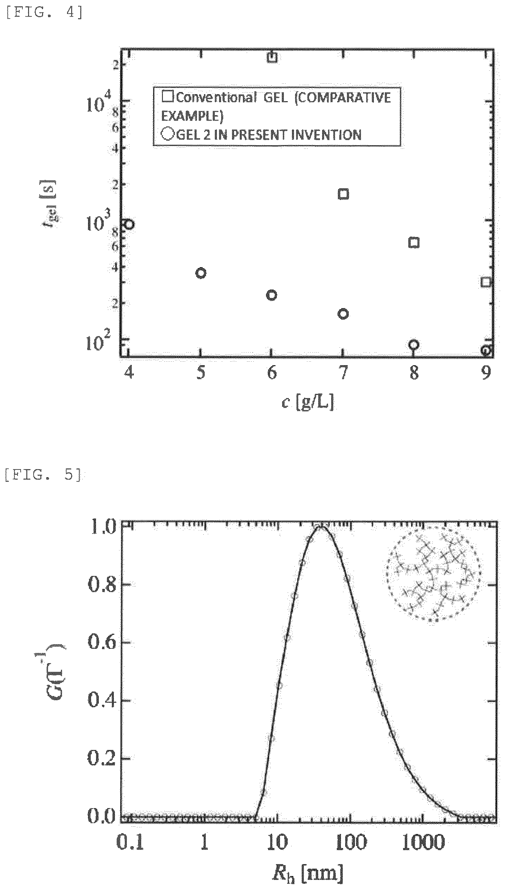

Using the gel precursor clusters 2 obtained in Example 2, a hydrogel was prepared in a similar manner. Each of the gel precursor cluster including excess SHPEG (10g/L; r = 0.37) and the gel precursor cluster including excess MAPEG (10 g/L; r = 0.63) was diluted to 6 g/L with a citrate buffer containing NaCl, and those gel precursor clusters were mixed in equal amount. Similarly to Fig. 3, Fig. 4 illustrates reaction time when gelation was performed by changing the concentrations of the gel precursor clusters. In the drawing, ○ represents Example in which the hydrogel of the present invention gelled from the gel precursor clusters, and □ represents Comparative Example in which a hydrogel gelled directly from polymer units by a conventional method without using gel precursor clusters. Particularly, at a concentration in polymer content as low as about 7 g/L, the gel precursor clusters of the present invention gelled within 3 minutes. This result indicates that, in vitreous surgeries, the gel precursor clusters can be injected intraocularly and allowed to gel in vivo.

### Example 4

### Physical Properties of Gel Precursor Clusters

### 1. Size of Gel Precursor Clusters

Fig. 5 illustrates a measurement result on a size distribution of the gel precursor clusters 1 synthesized in Example 2. The particle diameter (nm) of the gel precursor clusters is taken along the abscissa, represented by Rh, and the function of characteristic relaxation time distribution is taken along the ordinate, represented by G (Γ⁻¹). The result shows that the particle diameter of the gel precursor clusters is several hundred nm, and the particle diameter is mostly about 100 nm. Even the gel precursor clusters 2 synthesized in Example 2 yielded a substantially similar result.

### 2. Elastic Modulus

In regard to the gel precursor clusters 1 in the solution, dynamic viscoelasticity was measured with a rheometer (Physica MCR 501, manufactured by Anton Paar) to calculate a storage elastic modulus G' and a loss elastic modulus G". As a result, G" at 1 Hz was in the range of 0.1 < G" < 100 Pa and G' < G" < 100 G'. From this result, it is confirmed that the gel precursor clusters 1 obtained in Example 2 have a structure that has not reached the gelation criticality. Even the gel precursor clusters 2 synthesized in Example 2 yielded a substantially similar result.

### 3. Fractal Dimension

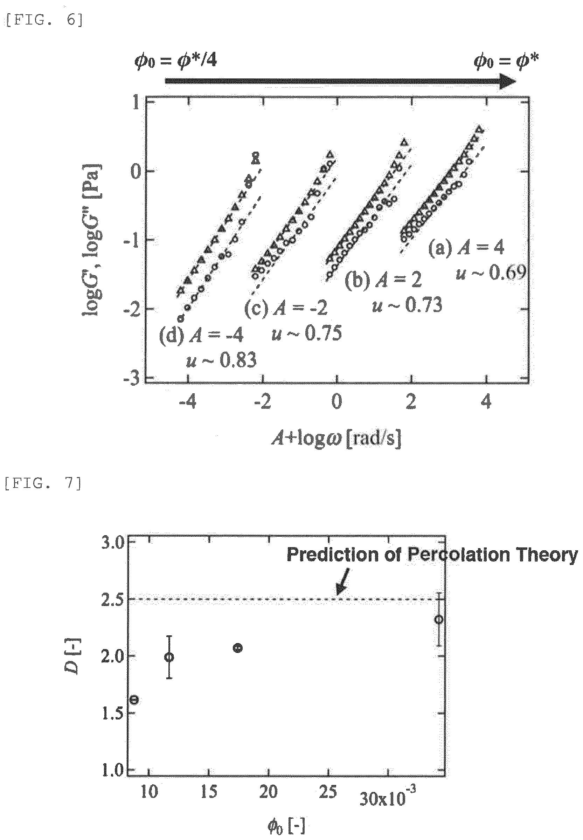

Fig. 6 illustrates a measurement result on dynamic viscosity characteristics at a gelation critical point in a case where initial concentrations of various polymer units are used in regard to the gel precursor clusters 1 obtained in Example 2. In Fig. 6, the storage elastic modulus G' (○ in the drawing) and the loss elastic modulus G" (Δ in the drawing) are taken along the ordinate, and the frequency is taken along the abscissa. The alphabets (a)-(d) shows conditions of each initial concentration. As illustrated in Fig. 6, the lower the initial concentration, the more the power law of G' and G" increases. Based on this result, the fractal dimension of the gel precursor clusters was calculated by the dynamic scaling theory. Fig. 7 illustrates the result. In Fig. 7, the fractal dimension is taken along the ordinate, and the initial concentration is taken along the abscissa. As can be seen in Fig. 7, the lower the concentration, the more the fractal dimension D deviates downward from the theoretical predicted value (dotted line in the drawing), which indicates that a more sparser structure is formed. Even the gel precursor clusters 2 synthesized in Example 2 yielded a substantially similar result.

### Example 5

### Physical Properties of Hydrogel

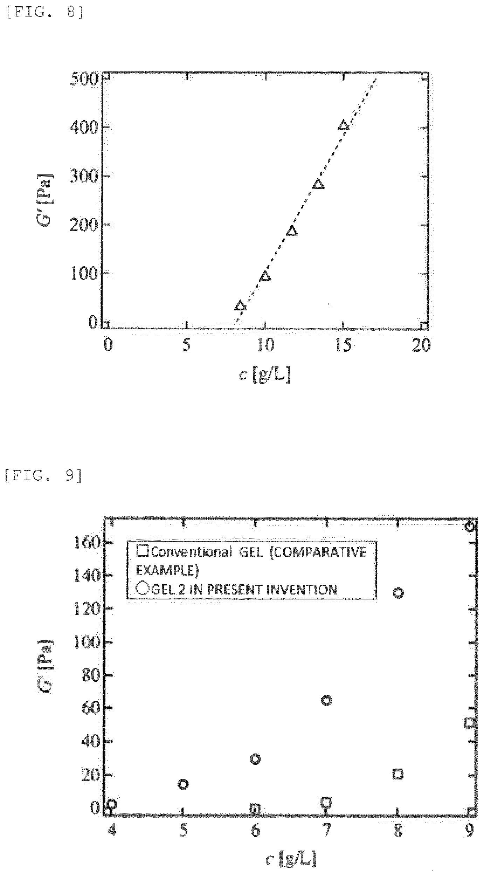

Polymer concentration dependency of the elastic modulus in the hydrogel 1 obtained in Example 3 was also measured. As illustrated in Fig. 8, the result shows that the elastic modulus is proportional to the polymer content in a region with a low elastic modulus which has a concentration as low as 20 g/L and a storage elastic modulus G' less than 400 Pa. This result verifies that it is possible to control the elastic modulus of the gel even in the region with the low elastic modulus by a method of gelation from the gel precursor clusters.

Similarly, polymer concentration dependency of the elastic modulus in the hydrogel 2 obtained in Example 3 was also measured. Fig. 9 illustrates the result. In the drawing, ○ represents Example in which the hydrogel of the present invention gelled from the gel precursor clusters, and □ represents Comparative Example in which a hydrogel gelled directly from polymer units by a conventional method without using gel precursor clusters. In either case, the hydrogel of the present invention shows a higher elastic modulus, indicating that an effective three-dimensional network is formed. The elastic modulus of the hydrogel was within the range of elastic moduli of vitreous body (10° to 10¹ Pa) and crystalline lens (10² to 10³ Pa).

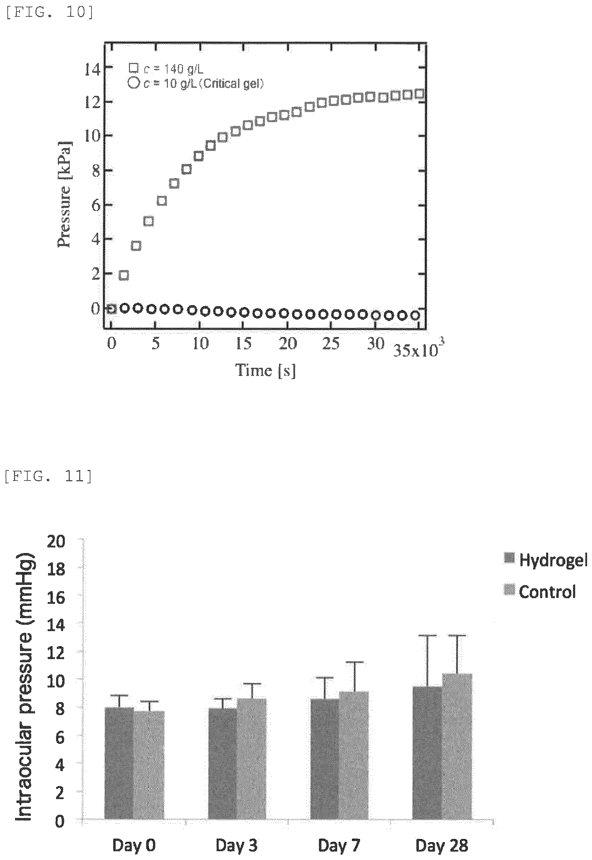

Fig. 10 illustrates the measurement result on temporal change of swelling pressure in regard to the hydrogel 2 obtained in Example 3. In the drawing, ○ represents Example in which the hydrogel of the present invention gelled from the gel precursor clusters (polymer concentration 10 g/L), and □ represents Comparative Example in which a hydrogel gelled directly from polymer units by a conventional method without using the gel precursor clusters (polymer concentration 140 g/L). As illustrated in Fig. 10, the hydrogel in Comparative Example reached equilibrium at the pressure of 12 kPa with time, but the hydrogel of the present invention was constant at about 0.19 kPa. This result indicates that even when the hydrogel of the present invention is intraocularly applied and stays for a long time, the hydrogel causes no cataracts or the like due to an increase in intraocular pressure, which indicates that the hydrogel can be used as a long-term stable tamponade material or a synthetic vitreous body.

### Example 6

### Intraocular Injection Test

The gel precursor clusters 2 obtained in Example 2 was intraocularly injected into a rabbit, and the hydrogel 2 of Example 3 was formed so as to verify the effect as a synthetic vitreous body in the following manner.

### 1. Measurement of Intraocular Pressure

A mixed solution of the gel precursor clusters including excess SHPEG (10 g/L; r = 0.37) prepared in Example 2 and the gel precursor clusters including excess MAPEG (10 g/L; r = 0.63) was injected with an injection needle (27 gauge) to the left eye of a rabbit whose vitreous body was resected (the number of samples: 7). Gelation inside the vitreous cavity was observed.

Fig. 11 illustrates results measured by a tonometer on intraocular pressure before surgery, and 3 days, 7 days, and 28 days after surgery. There was no significant difference in intraocular pressure between a group injected with the gel and a control group injected with a balanced salt solution (BBS).

### 2. Slit Lamp Test and Indirect Eyeground Test

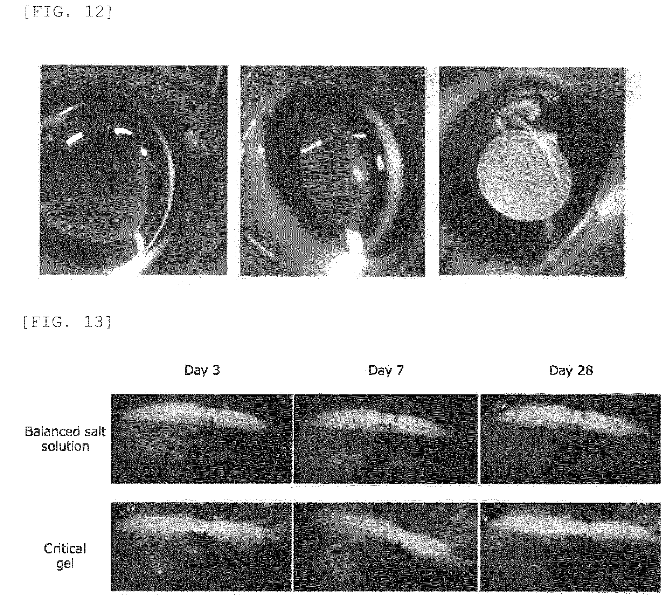

Fig. 12 illustrates the results of a slit lamp test performed on the eye of the rabbit into which the gel precursor clusters 2 obtained in Example 2 was injected and in which the hydrogel was formed intraocularly. Fig. 12 shows images of an anterior eye. The image on the left side shows the anterior eye injected with the hydrogel according to Example of the present invention; the image in the center shows the anterior eye injected with the balanced salt solution; and the image on the right side shows the anterior eye injected with the hydrogel according to Comparative Example. Herein, the hydrogel of Comparative Example is one in which TAPEG + TNPEG is mixed at a ratio of 1 : 1 (concentration: 100 g/L) and directly gelled without forming gel precursor clusters. As a result, clouding of the vitreous body due to inflammation was observed in the image on the right side that shows the anterior eye injected with the hydrogel according to Comparative Example, whereas, no inflammation was observed in the image on the left side that shows the anterior eye in Example of the present invention, and in the image in the center that shows the anterior eye of the control group.

In addition, similar tests on eyeground was performed, but no inflammation, hemorrhage, or retinal detachment was observed in Example of the present invention even after 3 days, 7 days, and 28 days after surgery (Fig. 13; the upper row shows images of the control group with the eye injected with the balanced salt solution, and the lower row shows images according to Example of the present invention in which the gel precursor clusters 2 obtained in Example 2 were injected and the hydrogel was formed intraocularly). Furthermore, in regard to optical coherence tomography (OCT), neither retinal detachment nor retinal edema were observed in Example of the present invention even after 28 days after surgery.

## Claims

1. A gel material for ophthalmic treatment including a hydrogel in which a gel precursor cluster crosslinks to form a three-dimensional network,
wherein the gel precursor cluster has a structure with a crosslinked monomer unit or a crosslinked polymer unit present at a concentration less than a critical gelation concentration, and the gel precursor cluster has a relationship of G' < G" where G' represents a storage elastic modulus and G" represents a loss elastic modulus, and
wherein the hydrogel has a polymer content of 50 g/L or less, a storage elastic modulus G' of 1 to 10,000 Pa at a frequency of 1 Hz, and a fractal dimension of 1.5 to 2.5.

2. The gel material for ophthalmic treatment according to claim 1, wherein the hydrogel has a loss elastic modulus G" of 1 to 100 Pa.

3. The gel material for ophthalmic treatment according to claim 1 or 2, wherein, in an aqueous solution, the hydrogel has a swelling pressure of 0.1 to 5 kPa and a swelling degree in a range where the volume of the hydrogel in a temperature of 30 to 40°C changes from 90 to 500% of the volume at the time of gel formation.

4. The gel material for ophthalmic treatment according to any one of claims 1 to 3, wherein the monomer unit has a vinyl skeleton, or the polymer unit has a polyethylene glycol skeleton or a polyvinyl skeleton.

5. The gel material for ophthalmic treatment according to any one of claims 1 to 4, wherein the gel precursor cluster includes a first polymer unit having one or more nucleophilic functional groups in a side chain or at an end and a second polymer unit having one or more electrophilic functional groups in a side chain or at an end.

6. The gel material for ophthalmic treatment according to claim 5, wherein the nucleophilic functional group is selected from the group consisting of an amino group, -SH, and -CO₂PhNO₂, and the electrophilic functional group is selected from the group consisting of N-hydroxy-succinimidyl (NHS) group, a sulfosuccinimidyl group, a maleimidyl group, a phthalimidyl group, an imidazoyl group, an acryloyl group, and a nitrophenyl group.

7. The gel material for ophthalmic treatment according to claim 5, wherein the nucleophilic functional group is - SH, and the electrophilic functional group is a maleimidyl group.

8. The gel material for ophthalmic treatment according to claim 5,
wherein the gel precursor cluster includes a first gel precursor cluster and a second gel precursor cluster,
wherein the first gel precursor cluster has a content of the first polymer unit higher than a content of the second polymer unit, and
the second gel precursor cluster has a content of the second polymer unit higher than a content of the first polymer unit.

9. The gel material for ophthalmic treatment according to any one of claims 1 to 8, wherein the loss elastic modulus G" of the gel precursor cluster is in the range of 0.005 to 5 Pa at a frequency of 1 Hz.

10. The gel material for ophthalmic treatment according to any one of claims 1 to 9, wherein the gel precursor cluster has a fractal dimension of 1.5 to 2.5.

11. The gel material for ophthalmic treatment according to any one of claims 1 to 10, wherein the gel precursor cluster has a diameter in the range of 10 to 1000 nm.

12. The gel material for ophthalmic treatment according to any one of claims 1 to 11, wherein the gel material is used as a vitreous injectant.

13. The gel material for ophthalmic treatment according to any one of claims 1 to 11, wherein the gel material is used as a synthetic vitreous body.

14. A polymer composition for ophthalmic treatment including a gel precursor cluster, wherein the gel precursor cluster has a structure with a crosslinked monomer unit or a crosslinked polymer unit at a concentration less than a critical gelation concentration, and the gel precursor cluster has a relationship of G' < G" where G' represents a storage elastic modulus and G" represents a loss elastic modulus.

15. The polymer composition for ophthalmic treatment according to claim 14, wherein the monomer unit has a vinyl skeleton, or the polymer unit has a polyethylene glycol skeleton or a polyvinyl skeleton.

16. The polymer composition for ophthalmic treatment according to claim 14 or 15, wherein the gel precursor cluster includes a first polymer unit having one or more nucleophilic functional groups in a side chain or at an end and a second polymer unit having one or more electrophilic functional groups in a side chain or at an end.

17. The polymer composition for ophthalmic treatment according to claim 16, wherein the nucleophilic functional group is selected from the group consisting of an amino group, -SH, and -CO₂PhNO₂, and the electrophilic functional group is selected from the group consisting of N-hydroxy-succinimidyl (NHS) group, a sulfosuccinimidyl group, a maleimidyl group, a phthalimidyl group, an imidazoyl group, an acryloyl group, and a nitrophenyl group.

18. The polymer composition for ophthalmic treatment according to claim 16, wherein the nucleophilic functional group is -SH, and the electrophilic functional group is a maleimidyl group.

19. The polymer composition for ophthalmic treatment according to 16, wherein the gel precursor cluster has a content of the first polymer unit higher than a content of the second polymer unit, or the gel precursor cluster has a content of the second polymer unit higher than a content of the first polymer unit.

20. The polymer composition for ophthalmic treatment according to any one of claims 14 to 19, wherein the gel precursor cluster has the loss elastic modulus G" in the range of 0.005 to 5 Pa at a frequency of 1 Hz.

21. The polymer composition for ophthalmic treatment according to any one of claims 14 to 20, wherein the gel precursor cluster has a fractal dimension of 1.5 to 2.5.

22. The polymer composition for ophthalmic treatment according to any one of claims 14 to 21, wherein the gel precursor cluster has a diameter in the range of 10 to 1000 nm.

23. A kit comprising the polymer composition for ophthalmic treatment according to any one of claims 16 to 22.

24. The kit according to claim 23, further comprising a crosslinking agent.

25. The kit according to claim 23,
wherein the gel precursor cluster includes the first polymer unit having one or more nucleophilic functional groups in a side chain or at an end and the second polymer unit having one or more electrophilic functional groups in a side chain or at an end, and the gel precursor cluster stores the following two types of polymer compositions (a) and (b) without mixing those polymer compositions:
(a) a polymer composition including a first gel precursor cluster that has a content of the first polymer unit higher than a content of the second polymer unit; and
(b) a polymer composition including a second gel precursor cluster that has a content of the second polymer unit higher than a content of the first polymer unit.
